(19) Europäisches Patentamt
European Patent Office
Office européen des brevets

(11) **EP 2 479 575 B1**

(12) **EUROPEAN PATENT SPECIFICATION**

(45) Date of publication and mention of the grant of the patent:
**20.04.2016 Bulletin 2016/16**

(21) Application number: **10816988.9**

(22) Date of filing: **04.08.2010**

(51) Int Cl.:
*G01N 33/66* (2006.01)  *A61B 5/145* (2006.01)
*G01N 21/78* (2006.01)  *G01N 33/483* (2006.01)
*A61B 5/1455* (2006.01)  *G01N 27/327* (2006.01)
*G01N 21/84* (2006.01)  *G01N 33/49* (2006.01)

(86) International application number:
**PCT/JP2010/063197**

(87) International publication number:
**WO 2011/033875 (24.03.2011 Gazette 2011/12)**

(54) **BLOOD GLUCOSE METER**

BLUTZUCKERMESSGERÄT

GLUCOMÈTRE SANGUIN

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **17.09.2009 JP 2009216140**

(43) Date of publication of application:
**25.07.2012 Bulletin 2012/30**

(73) Proprietor: **Terumo Kabushiki Kaisha Tokyo 151-0072 (JP)**

(72) Inventor: **MOMOKI Hideyuki
Nakakoma-gun
Yamanashi 409-3853 (JP)**

(74) Representative: **TBK
Bavariaring 4-6
80336 München (DE)**

(56) References cited:
**EP-A1- 2 479 576      WO-A1-2007/116675
CA-A1- 2 241 964      JP-A- 8 114 538
JP-A- 2002 168 862    JP-A- 2004 294 107
JP-A- 2008 203 114    JP-A- 2008 232 664
US-A1- 2008 309 939**

## Description

## Technical Field

The present invention relates to a technique preferably applied to a blood glucose meter.

More particularly, the present invention relates to a blood glucose meter which ensures required measurement accuracy while reducing power consumption as much as possible.

## Background Art

As is well known, diabetes mellitus is caused by abnormal secretion of insulin from the pancreas or decreased insulin sensitivity. For a type I diabetic patient whose pancreas does not secrete insulin, it is particularly necessary to measure blood glucose level of the patient before meals, and administer insulin to the patient according to the measured level.

Conventionally, in order to easily measure blood glucose level by patient himself/herself or by his/her family member at home, a miniaturized blood glucose measuring device (referred to as "blood glucose meter" hereinafter) for self-measurement of blood glucose level has been developed, manufactured and marketed by the applicant of the present invention. Further, a blood glucose meter having various management functions and capable of dealing with a plurality of patients is being developed for hospital wards by the applicant of the present invention.

A blood glucose meter is a device for measuring blood glucose level based on a principle that a biochemical reaction is caused by contacting blood with a reagent, and thereby the glucose value is converted into color density or electrical signal. It will take time before the biochemical reaction becomes stable.

However, since there are a lot of patients to be treated in a hospital ward, the blood glucose measurement has to be performed quickly. In other words, there is little time to wait until the biochemical reaction becomes stable. To solve this problem, it is considered to more quickly obtain the blood glucose value based on a transient response of the biochemical reaction during the reaction, instead of waiting until the biochemical reaction becomes stable. For example, as shown in Patent document 1 and Patent document 2, there are known methods for predicting the reaction end point based on a predetermined calculation formula or the like.

Patent document 1: Japanese Unexamined Patent Application Publication No. 2004-144750.

Patent document 2: Japanese Unexamined Patent Application Publication No. 2006-71421.

*Further prior art can be found in document* US 2008/309939 A1*, disclosing a body fluid constitutents measurement device with the features known from the preamble of the appended independet claim 1, and in particular with a light-emitting element that emits light* onto a test paper onto which body fluid is spotted, a light receiving element which receives reflected light of the light emitted by said light-emitting element, a temperature measurement unit which measures the ambient temperature in the vicinity of said light-emitting element, a determination unit which determines conditions of light emission based on said temperature measured at said temperature measurement unit in order to stabilize light intensity of said light-emitting element, and with a driving control unit which controls driving of said light-emitting element based on the conditions of light emission. The device starts measurement of body fluid constituents after the light intensity of light emitted by said light-emitting element has been stabilized.

*Further prior art can be found in document* CA 2 241 964 A1*, disclosing a medical diagnostic apparatus with a sleep mode.*

## Disclosure of the Invention

[Problems to be Solved by the Invention]

In the case where the blood glucose value is obtained based on a transient response of the biochemical reaction, strict time management is necessary. A predetermined measurement time has to be accurately measured from the start of the biochemical reaction, i.e., the moment when the blood contacts the reagent.

In the case where the glucose value is obtained by observing the color density, a light-emitting element such as a LED or the like is used to radiate light onto a test paper in which a reagent is included, the light reflected at the test paper is detected by a light-receiving element such as a photodiode or the like, and the detected light is converted into digital data by an A/D converter. If the frequency of the sampling clock supplied to the A/D converter is low, the moment when the blood contacts the reagent can not be correctly detected. Thus, in order to correctly detect the start of the reaction, the frequency of the sampling clock needs to be set to a sufficiently high value. However, for a blood glucose meter which is a portable electronic device, since the A/D converter is a component that consumes a large mount of power, it is preferred that the frequency of the sampling clock is set as low as possible.

The present invention has been made in view of the above problems, and it is an object of the present invention to provide a blood glucose meter in which good balance between measurement accuracy and low power consumption can be achieved by performing measurement with a frequency of sampling clock that ensures sufficient accuracy as well as with minimum time.

[Means for Solving the Problems]

To solve the aforesaid problems, a blood glucose meter according to an aspect of the present invention comprises: a blood glucose measuring section adapted to, in a state where a measuring tip is mounted, *detect* blood *spotted* onto the measuring tip, and output a signal corresponding to glucose level of the blood; an A/D converter adapted to convert the signal into digital data; a blood spotting judgment section adapted to judge whether or not the blood has been spotted onto the measuring tip based on the digital data; a measurement processing section adapted to acquire a blood glucose value based on the digital data; and a clock generator adapted to generate a clock for acquiring the digital data from the A/D converter, wherein the period of the clock is *equal to* or less than a value obtained by dividing the required resolution by the slope of a blood glucose reaction curve at a predetermined time.

The clock for acquiring the digital data from the A/D converter is determined based on the required resolution and the slope of the blood glucose measurement curve.

The clock generators are configured so that the sampling clock is suitably set according to the operating mode of the blood glucose meter. Particularly, the clock generators are configured so that the high-speed clock pulses are continuously generated in a mode that determines measurement start, and, from the time when the spotting is detected, the measurement operation of the timer and the like is started, and the mode is switched from continuously generating the clock pulses to intermittently generating the clock pulses.

[Advantages of the Invention]

According to the present invention, it is possible to provide a blood glucose meter in which good balance between measurement accuracy and low power consumption can be achieved by performing measurement with a frequency of sampling clock that ensures sufficient accuracy as well as with minimum time.

**Brief Description of Drawings**

FIG. 1 is a perspective view showing the appearance of a blood glucose meter according to an embodiment of the present invention;
FIG. 2 is a top view of the blood glucose meter according to the aforesaid embodiment of the present invention;
FIG. 3 is a schematic illustration of an optical measuring section;
FIG. 4 is an internal block diagram of the blood glucose meter;
FIG. 5 is a functional block diagram of the blood glucose meter;
FIG. 6 is a table showing states where a controller controls a low-speed clock generator or a high-speed clock generator;
FIG. 7 is a functional block diagram of the high-speed clock generator;
FIG. 8 is a functional block diagram of a low-speed clock generator;
FIGS. 9A, 9B, 9C and 9D are each a timing diagram showing clock pulses generated by the low-speed clock generator or the high-speed clock generator.
FIGS. 10A, 10B, 10C and 10D are each a timing diagram showing timing pulses or a sampling clock generated by a high-speed sequencer;
FIGS. 11A, 11B and 11C are each a timing diagram showing timing pulses or a sampling clock generated by a low-speed sequencer;
FIG. 12 is a graph of a blood glucose reaction curve which shows measurement value calculated using standard curve data when measuring blood glucose level, with the time course.

**Best Modes for Carrying Out the Invention**

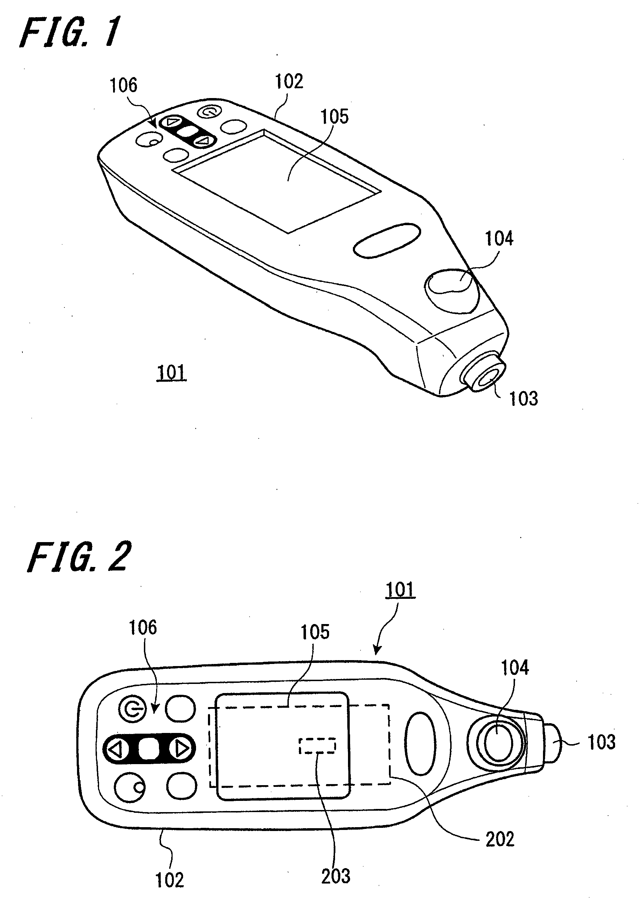

An embodiment of the present invention will be described below with reference to FIGS. 1 to 12.

FIG. 1 is a perspective view showing the appearance of a blood glucose meter according to the embodiment of the present invention.

FIG. 2 is a top view of the blood glucose meter according to the embodiment of the present invention.

A blood glucose meter 101 is a portable device for measuring blood glucose level. The blood glucose meter 101 is adapted to be operated by one hand of a doctor, a nurse or a patient himself/herself, just like a cellular phone, and therefore is formed so as to have a shape and weight suitable to be easily held by one hand.

The blood glucose meter 101 not only can measure blood glucose level but also, as additional functions, can perform other operations such as confirming the name and ID of a patient, storing measurement value data for each patient and, if necessary, confirming appropriate drug(s) to be administered for each patient.

The blood glucose meter 101 has a case 102, which is a slender container made of synthetic resin. An optical measuring section 103 for measuring blood glucose level or the like is provided at a tip end in the longitudinal direction of the case 102. The optical measuring section 103 is a cylindrical component made of metal. LED and photodiode (which are to be described later) are built into the optical measuring section 103, which is also referred to as "blood glucose measuring section".

The optical measuring section 103 is formed so that a blood glucose measuring tip (referred to as "measuring tip" hereinafter) can be mounted to and dismounted from the optical measuring section 103. The used measuring tip can be dismounted from the optical measuring section 103 by operating an eject lever 104.

Further, a display panel 105 which is a LCD for displaying the measurement result, the confirmation items and the like is arranged on the front face of the case 102. An operation panel 106 having a plurality of buttons is arranged beside the display panel 105.

Other components arranged in the blood glucose meter 101 include: a lithium-ion battery (not shown in the drawings) built into the case 102, a bar code reader unit (not shown) for reading bar code, an IrDA interface (not shown) for transmitting/receiving the patient data, the measured blood glucose data and the like. However, since these components do not directly relate to the present invention, the details thereof will not be described herein.

As shown in FIG. 2, although not invisible from the outside of the blood glucose meter 101, a circuit board 202 (which is a printed board) is built into the meter body. A known microcomputer is mounted on the circuit board 202. The microcomputer, which is powered by the lithium-ion battery, is adapted to receive an operating command signal from the operation panel 106, drive the LED arranged in the optical measuring section 103, perform predetermined blood glucose measurement through the photodiode, and display measurement result and the like on the display panel 105.

The basic blood glucose measuring mechanism of the blood glucose meter 101 is identical to that of the prior art, and therefore the outline will be briefly described below.

A measuring tip 308 is mounted on the optical measuring section 103 to suck the blood of the object person to be measured into the measuring tip 308. A test paper 511 formed of a porous membrane, such as a polyethersulfone membrane, is built into the measuring tip 308. Further, when the blood sucked into the measuring tip 308 reaches the test paper 511, the glucose in the blood will react with the reagent contained in the test paper 511 so as to develop a color. The chromogenic reaction will take a time from several seconds to over ten seconds, and the chromogenic reaction is affected according to the ambient temperature.

The light emitted by the LED (the light-emitting element) is radiated onto the test paper 511, and the light reflected from the test paper 511 is received by the photodiode (the light-receiving element). After a predetermined reaction time has elapsed, an analog intensity signal of the received light obtained from the light-receiving element is converted into a digital value, and thereafter the digital value is converted into the blood glucose value to be displayed on the display panel 105.

Incidentally, instead of being limited to the aforesaid optical measurement method in which the chromogenic reagent is used, the mechanism on the blood glucose measuring side may also be a mechanism conventionally used to measure blood glucose level, such as an electrochemical sensor method or the like.

As described above, when measuring blood glucose level, the reaction time of the reagent contained in the test paper 511 changes according to the ambient temperature. For this reason, reaction correction values with respect to the ambient temperature are stored in a ROM, which is a constituent element of the microcomputer inside of blood glucose meter 101. Further, a program of the microcomputer stored in the ROM is constituted so that the ambient temperature when measuring blood glucose level is detected to calculate suitable measurement value.

However, the correction value can not be correctly derived if the ambient temperature changes during the measurement. Thus, there is a very high risk that an incorrect blood glucose value might be derived. In other words, the ambient temperature must not change during the measurement. Obviously, if there was a change in ambient temperature immediately before the measurement, the blood glucose measurement processing can not be performed until the change is quieted down.

In order to detect that the ambient temperature of the blood glucose meter 101 is stable, the blood glucose meter 101 is provided with two temperature measuring elements.

One of the temperature measuring elements is an external temperature sensor for measuring the ambient air temperature (referred to as "external temperature" hereinafter). The external temperature sensor is disposed at a position separated from the central portion of the case of the blood glucose meter 101, and therefore is thermally independent from the case.

The other one of the temperature measuring elements is an internal temperature sensor for measuring the temperature inside the case (referred to as "internal temperature" hereinafter). The internal temperature sensor is disposed in the central portion of the case of the blood glucose meter 101.

If the two temperature sensors do not change even a predetermined period of time has elapsed and if difference between the two temperature sensors is small, then it can be judged that the whole case of the blood glucose meter 101 has "acclimatized" to the external temperature (i.e., it can be judged that the difference between the temperature outside the case of the blood glucose meter 101 and the temperature inside the case of the blood glucose meter 101 is small enough to correctly measure blood glucose level).

An internal temperature thermistor 203 (which is the internal temperature sensor) is mounted on the circuit board 202 in the same manner as other circuit components constituting the microcomputer and the like.

On the other hand, an external temperature thermistor 307 (which is the external temperature sensor) is arranged in the optical measuring section 103.

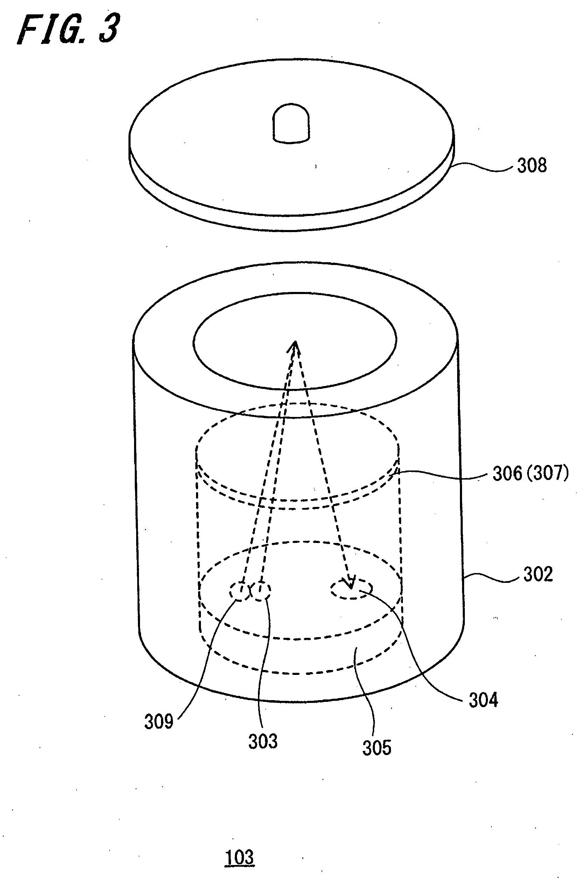

FIG. 3 is a schematic illustration of the optical measuring section 103.

The optical measuring section 103 includes a tube 302, a red LED 303, a photodiode 304, a base 305, a glass window 306 and a green LED 309, wherein the red LED 303, the photodiode 304, the base 305, the glass window 306 and the green LED 309 are housed in the tube 302.

Inside the tube 302, which is made of a metal such as a stainless steel or the like, the red LED 303, the green LED 309 and the photodiode 304 are mounted on the base 305.

For dust-proof purpose, the base 305 is shut off from the ambient air by the glass window 306, which is made of a thin glass plate. A platinum wire is printed on the glass window 306, the wire and the glass window constituting the external temperature thermistor 307.

A holding mechanism (not shown in the drawings) is incorporated into the tube 302, the holding mechanism being adapted to enable the measuring tip 308 to be mounted and dismounted. When the measuring tip 308 is mounted, the test paper 511 is disposed so as to face the glass window 306.

The red LED 303 radiates red light onto the test paper 511 provided in the measuring tip 308. The red LED 303 is driven to emit light in all operating modes (which are to be described later).

The green LED 309 radiates green light onto the test paper 511 provided in the measuring tip 308. The green LED 309 is driven to emit light in the operating mode(s) where high measuring accuracy is required, among a plurality of operating modes (which are to be described later).

An absorption wavelength specific to the dye generated by the reaction of the glucose with the reagent, for example 630 nm, is used as the emission wavelength of the red LED 303. An absorption wavelength specific to the hemoglobin contained in the blood, for example 520 nm, is used as the emission wavelength of the green LED 309. Hematocrit value is calculated based on the absorbance measured by the wavelength of the green LED 309, and the blood glucose value is calculated based on the hematocrit value and the absorbance measured by the wavelength of the red LED 303.

In order to quickly and suitably measure the external temperature, the external temperature thermistor 307 needs to have a small heat capacity. Thus, the glass plate configuring the external temperature thermistor 307 is formed to a size of 6 mm in diameter and 0.5 mm in thickness. The heat capacity of the glass plate is about 4 mJ/K.

[Hardware]

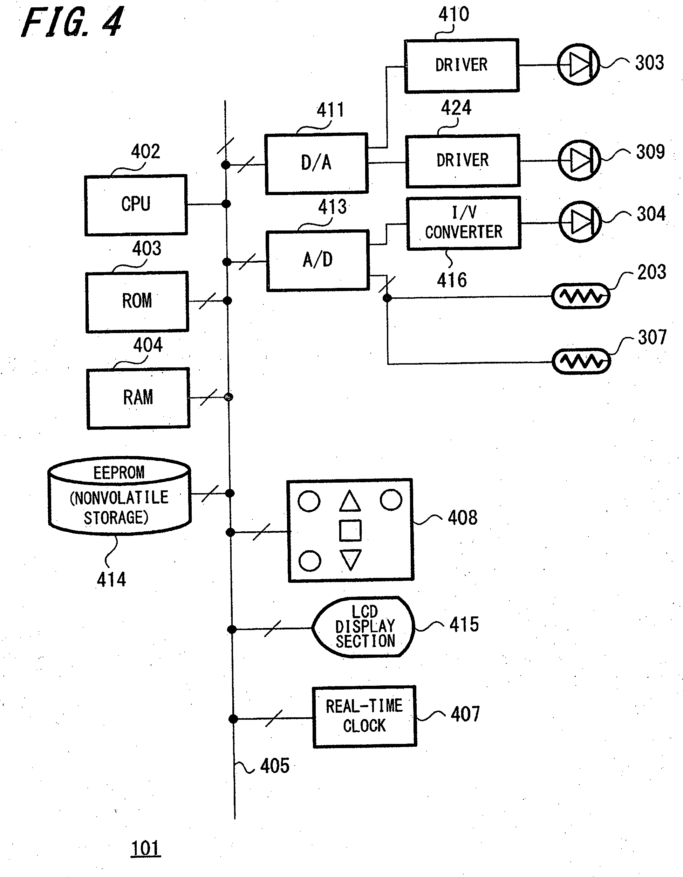

FIG. 4 is an internal block diagram of the blood glucose meter 101.

The blood glucose meter 101 is a system having a microcomputer, and includes a CPU 402, a ROM 403, a RAM 404, and a bus 405, wherein the bus 405 connects the CPU 402, the ROM 403 and the RAM 404. In addition to the aforesaid components, a component for mainly providing data input function and a component for mainly providing data output function are connected to the bus 405.

The optical measuring section 103 (which is important to the blood glucose meter 101) for acquiring blood glucose measurement data, the internal temperature thermistor 203 and the external temperature thermistor 307 both for acquiring temperature data, a real-time clock 407, and a button operating portion 408 (which is the operation panel 106) are provided to the component for providing the data input function of the blood glucose meter 101.

A driver 410 for driving the red LED 303 to emit light is connected to the red LED 303, which constitutes the optical measuring section 103.

A driver 424 for driving the green LED 309 to emit light is connected to the green LED 309, which constitutes the optical measuring section 103.

The driving of the driver 410 and the driving of the driver 424 are controlled by a D/A converter 411 in a time-sharing manner. The details about the time-sharing driving will be described later.

An A/D converter 413 is connected to the photodiode 304, which constitutes the optical measuring section 103, through an I/V converter 416.

Since it is necessary for the red LED 303 and the green LED 309 to radiate light of proper intensity to the test paper 511 of the measuring tip 308, the red LED 303 and the green LED 309 are controlled so that they emit light based on emission intensity data previously stored in a nonvolatile storage 414 (which is to be described later).

In other words, the program (which cause the microcomputer constituting the blood glucose meter 101 to operate) stored in the ROM 403 reads out the emission intensity data from the nonvolatile storage 414, converts, with the D/A converter 411, the emission intensity data into an analog voltage signal, and thereafter amplifies, with the driver 410, the power of the analog voltage signal to drive the red LED 303 to emit light. Similarly, the program reads out the emission intensity data from the nonvolatile storage 414, converts, with the D/A converter 411, the emission intensity data into an analog voltage signal, and thereafter amplifies, with the driver 424, the power of the analog voltage signal to drive the green LED 309 to emit light.

The light emitted by the red LED 303 and the green LED 309 is radiated onto the test paper 511 of the measuring tip 308, and the light reflected by the test paper 511 is detected by the photodiode 304.

The signal current of the photodiode 304, which varies according to the intensity of the light received by the photodiode 304, is converted into a signal voltage by the I/V converter 416, and further, the signal voltage is converted into numerical data by the A/D converter 413. The converted numerical data is recorded in predetermined regions of both the RAM 404 and the nonvolatile storage 414.

Further, the blood glucose meter 101 is provided with the internal temperature thermistor 203 and the external temperature thermistor 307, and the external temperature surrounding the blood glucose meter 101 and the internal temperature of the blood glucose meter 101 can be measured based on the changes in resistances of these thermistors. Similar to the photodiode 304, the resistances of the thermistors are converted into numeric values by the A/D converter 413, and the numeric data is recorded in predetermined regions of both the RAM 404 and the nonvolatile storage 414. Incidentally, since it is not necessary to measure the intensity of the received light and the ambient temperature at the same time, the A/D converter 413 is shared by the photodiode 304 and the thermistors in a time-sharing manner.

The real-time clock 407 is a known IC adapted to provide a date and time data output function, and is mounted on many microcomputers and personal computers as standard.

In the blood glucose meter 101 according to the embodiment of the present invention, since it is necessary to associate the patient data and blood glucose value with the date and time information at the time when the measurement is performed and store the data in the nonvolatile storage 414, the real-time clock 407 is provided.

A LCD display section 415 (which is the display panel 105) is provided as the component for providing the data output function of the blood glucose meter 101.

Various screens are displayed on the LCD display section 415 by the program stored in the ROM 403 and executed by the CPU 402.

Among the components constituting the microcomputer in the blood glucose meter 101, the nonvolatile storage 414 comprising an EEPROM, is adapted to provide data storage function, in addition to the data input/output function. Patient information, setting data of the blood glucose meter 101, accuracy test data and the like are stored in the nonvolatile storage 414. The data stored in the nonvolatile storage 414 is exchanged with an external device through an infrared interface, a wireless interface or the like (these are not shown in the drawings).

[Software]

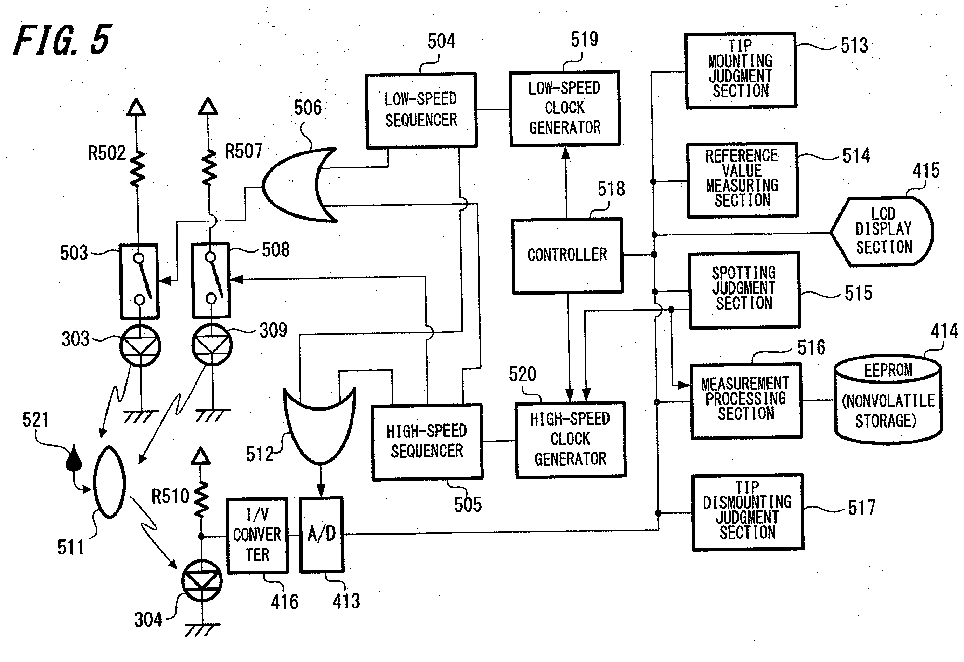

FIG. 5 is a functional block diagram of the blood glucose meter 101. FIG. 5 focuses on the functions provided by the microcomputer.

A power source voltage is applied to the red LED 303 through a voltage-dividing resistor R502 and a switch 503.

The on/off operation of the switch 503 is controlled by an emission control signal outputted by a low-speed sequencer 504 and an emission control signal outputted by a high-speed sequencer 505. An OR gate 506 collectively provides the emission control signal outputted by the low-speed sequencer 504 and the emission control signal outputted by the high-speed sequencer 505 to the switch 503. In other words, the emission of the red LED 303 is controlled by the low-speed sequencer 504 and the high-speed sequencer 505.

Similarly, the power source voltage is applied to the green LED 309 through a voltage-dividing resistor R507 and a switch 508.

The on/off operation of the switch 508 is controlled by the emission control signal outputted by high-speed sequencer 505. In other words, unlike the red LED 303, the emission of the green LED 309 is controlled only by the high-speed sequencer 505.

Incidentally, the switch 503 is actually equivalent to the D/A converter 411 and the driver 410. Similarly, the switch 508 is actually equivalent to the D/A converter 411 and the driver 424. Incidentally, the switch 503 and the switch 508 not only simply control on/off operation, but resistance of the drivers is controlled by the aid of the microcomputer.

Due to space limitations and for convenience of description, current control will not be described herein.

The light emitted by the red LED 303 and the green LED 309 is radiated onto the test paper 511 of the measuring tip 308, and the light reflected by the test paper 511 is detected by the photodiode 304.

The power source voltage is applied to the anode of the photodiode 304 through the voltage-dividing resistor R510. When the photodiode 304 receives the reflected light of the red LED 303 or the green LED 309 reflected from the test paper 511 of the measuring tip 308, the signal current of the photodiode 304 will change. The signal current is passed through the I/V converter 416 and converted into digital data by the A/D converter 413.

The I/V converter 416 converts the signal current of the photodiode 304 into a voltage, and the A/D converter 413 converts the voltage into digital data in accordance with the sampling clock outputted from either the low-speed sequencer 504 or the high-speed sequencer 505 through an OR gate 512.

The digital data outputted from the A/D converter 413 is inputted to a tip mounting judgment section 513, a reference value measuring section 514, a blood spotting judgment section 515, a measurement processing section 516 and a tip dismounting judgment section 517.

A controller 518 judges the current operating mode, and selectively controls the drive of the tip mounting judgment section 513, the reference value measuring section 514, the blood spotting judgment section 515, the measurement processing section 516 and the tip dismounting judgment section 517 according to the operating mode.

In the original state of the blood glucose meter 101, the controller 518 causes the tip mounting judgment section 513 to operate.

The tip mounting judgment section 513 is a program function (subroutine or function) that judges, upon receiving the digital data outputted from the A/D converter 413, whether the measuring tip 308 has been mounted on the optical measuring section 103.

When judging that the measuring tip 308 has been mounted on the optical measuring section 103, the tip mounting judgment section 513 reports that effect to the controller 518.

Incidentally, while the tip mounting judgment section 513 is in operation, the controller 518 controls a low-speed clock generator 519 to only drive the red LED 303 to emit light.

When the tip mounting judgment section 513 reports to the controller 518 that it is judged that the measuring tip 308 has been mounted on the optical measuring section 103, the controller 518 then causes the reference value measuring section 514 to operate.

Similar to the tip mounting judgment section 513, the reference value measuring section 514 is also a program function.

The reference value measuring section 514 radiates the light of the red LED 303 and the green LED 309 onto the test paper 511 of the measuring tip 308 mounted on the optical measuring section 103. Further, upon receiving the digital data outputted from the A/D converter 413, the reference value measuring, section 514 acquires the data of the reflected light in the state before the blood has been spotted onto the test paper 511 (i.e., the initial state), and stores the acquired data in the RAM 404.

After the data of the reflected light in the initial state has been stored in the RAM 404, the reference value' measuring section 514 reports to the controller 518 that the operation has been completed.

Incidentally, while the reference value measuring section 514 is in operation, the controller 518 controls a high-speed clock generator 520 to drive the red LED 303 and the green LED 309 to emit light.

After the reference value measuring section 514 has reported to the controller 518 that the reflected light data of the initial state has been stored in the RAM 404, the controller 518 causes the blood spotting judgment section 515 to operate.

Similar to the tip mounting judgment section 513 and the reference value measuring section 514, the blood spotting judgment section 515 is also a program function.

The blood spotting judgment section 515 radiates the light of the red LED 303 and the green LED 309 onto the test paper 511 of the measuring tip 308 mounted on the optical measuring section 103. Further, by receiving the digital data outputted from the A/D converter 413, the blood spotting judgment section 515 judges whether or not the blood has been spotted onto the test paper 511 based on the change of the amount of the reflected light.

If it is judged that the blood has been spotted onto the test paper 511, the blood spotting judgment section 515 reports that effect to the controller 518, and outputs a new control signal to both the high-speed clock generator 520 and the measurement processing section 516.

Incidentally, while the blood spotting judgment section 515 is in operation, the controller 518 controls the high-speed clock generator 520 to drive the red LED 303 and the green LED 309 to emit light.

Upon receiving the control signal outputted by the blood spotting judgment section 515 and triggered by the judgment that the blood has been spotted onto the test paper 511, the measurement processing section 516 starts the measurement processing.

Similar to the tip mounting judgment section 513, the reference value measuring section 514 and the blood spotting judgment section 515, the measurement processing section 516 is also a program function.

The measurement processing section 516 is started by the control signal outputted from the blood spotting judgment section 515. When the measurement processing section 516 is started, a timer (not shown in the drawings) provided therein is operated to count a predetermined time. The predetermined time is, for example, 9 seconds. The measurement processing section 516 radiates the light of the red LED 303 and the green LED 309 onto the test paper 511 of the measuring tip 308 mounted on the optical measuring section 103 until the timer completes counting. When the timer has completed counting, the measurement processing section 516 calculates the blood glucose value based on the digital data outputted from the A/D converter 413. The measurement processing section 516 displays the calculated blood glucose value onto the LCD display section, and stores the blood glucose value in the nonvolatile storage. Further, the measurement processing section 516 reports to the controller 518 that the processing is terminated.

Incidentally, while the measurement processing section 516 is in operation, the controller 518 controls the high-speed clock generator 520 to drive the red LED 303 and the green LED 309 to emit light.

After the measurement processing section 516 has reported to the controller 518 that the measurement processing is completed, the controller 518 causes the tip dismounting judgment section 517 to operate.

Similar to the tip mounting judgment section 513, the reference value measuring section 514, the blood spotting judgment section 515 and the measurement processing section 516, the tip dismounting judgment section 517 is also a program function.

Further, upon receiving the digital data outputted from the A/D converter 413, the tip dismounting judgment section 517 judges whether or not the measuring tip 308 is dismounted from the optical measuring section 103 based on the change of the amount of the reflected light.

When judging that the measuring tip 308 is dismounted from the optical measuring section 103, the tip dismounting judgment section 517 reports that effect to the controller 518.

Incidentally, while the tip dismounting judgment section 517 is in operation, the controller 518 controls the low-speed clock generator to only drive the red LED 303 to emit light.

The controller 518 controls the operations of the tip mounting judgment section 513, the reference value measuring section 514, the blood spotting judgment section 515, the measurement processing section 516 and the tip dismounting judgment section 517, and performs proper LED emission control while these components are in operation.

To be specific, while causing the tip mounting judgment section 513 and the tip dismounting judgment section 517 to operate, the controller 518 controls the low-speed clock generator 519 to output a clock for causing the red LED 303 and the A/D converter 413 to operate from the low-speed sequencer 504.

While causing the reference value measuring section 514, the blood spotting judgment section 515 and the measurement processing section 516 to operate, the controller 518 controls the high-speed clock generator 520 to output a clock for causing the red LED 303, the green LED 309 and the A/D converter 413 to operate from the high-speed sequencer 505.

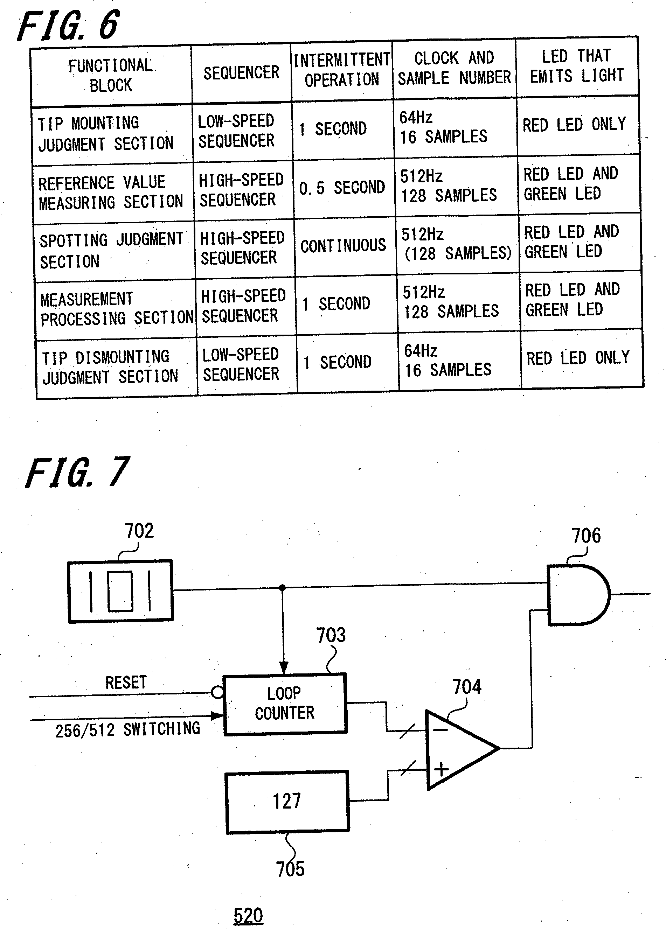

FIG. 6 is a table showing states where the controller 518 controls the low-speed clock generator 519 or the high-speed clock generator 520.

The controller 518 first causes the tip mounting judgment section 513 to operate, and controls the low-speed clock generator 519. The low-speed clock generator 519 generates 16 samples of clock pulses every 1 second with a 64 Hz clock, and provides the generated clock pulses to the low-speed sequencer 504. In response to the inputted clock, the low-speed sequencer 504 generates pulses for controlling the emission of the red LED 303 and a sampling clock for controlling the operation of the A/D converter 413.

Upon receiving the 16 samples of data from the A/D converter 413, the tip mounting judgment section 513 calculates an average value. The tip mounting judgment section 513 compares the average value with a predetermined threshold to judge whether or not the measuring tip 308 is mounted on the optical measuring section 103.

The controller 518 then causes the reference value measuring section 514 to operate, and controls the high-speed clock generator 520. The high-speed clock generator 520 generates 128 samples of clock pulses every 0.5 seconds with a 512 Hz clock, and provides the generated clock pulses to the high-speed sequencer 505. In response to the inputted clock, the high-speed sequencer 505 generates pulses for controlling the emission of the red LED 303, pulses for controlling the emission of the green LED 309, and a sampling clock for controlling the operation of the A/D converter 413.

Upon receiving the 128 samples of data from the A/D converter 413, the reference value measuring section 514 calculates an average value. The reference value measuring section 514 compares the average value with a predetermined threshold to obtain the reference value in the state before the blood has been spotted onto the test paper 511 of the measuring tip 308, and stores the obtained data in the RAM 404.

The controller 518 then causes the blood spotting judgment section 515 to operate, and controls the high-speed clock generator 520. The high-speed clock generator 520 continuously, generates clock pulses with the 512 Hz clock, and provides the generated clock pulses to the high-speed sequencer 505. In response to the inputted clock, the high-speed sequencer 505 generates pulses for controlling the emission of the red LED 303, pulses for controlling the emission of the green LED 309, and a sampling clock for controlling the operation of the A/D converter 413.

Upon receiving the data from the A/D converter 413, the blood spotting judgment section 515 continuously calculates a moving average value of the 128 samples. The blood spotting judgment section 515 compares the moving average value with a predetermined threshold to judge whether or not the blood has been spotted onto the test paper 511 of the measuring tip 308.

If it is judged that the blood has been spotted onto the test paper 511, the blood spotting judgment section 515 outputs a control signal to both the measurement processing section 516 and the high-speed clock generator 520.

Upon receiving the control signal from the blood spotting judgment section 515, the high-speed clock generator 520 switches from the previous state where clock pulses are continuously generated with the 512 Hz clock to a state where 128 samples of clock pulses are intermittently generated every 1 second with the 512 Hz clock. In response to the inputted clock, the high-speed sequencer 505 generates pulses for controlling the emission of the red LED 303, pulses for controlling the emission of the green LED 309, and a sampling clock for controlling the operation of the A/D converter 413.

On the other hand, upon receiving the control signal from the blood spotting judgment section 515, the measurement processing section 516 starts the measurement processing. Thereafter, upon receiving 128 samples of data from the A/D converter 413, the measurement processing section 516 calculates an average value. The average value is sequentially stored in the RAM 404 until a predetermined time has elapsed. Finally, the measurement processing section 516 calculates the blood glucose value by using standard curve data stored in the nonvolatile storage 414.

Thereafter, while the tip dismounting judgment section 517 is in operation, the controller 518 controls the low-speed clock generator 519. The low-speed clock generator 519 generates 16 samples of clock pulses every 1 second with a 64 Hz clock, and provides the generated clock pulses to the low-speed sequencer 504. In response to the inputted clock, the low-speed sequencer 504 generates pulses for controlling the emission of the red LED 303 and a sampling clock for controlling the operation of the A/D converter 413.

Upon receiving the 16 samples of data from the A/D converter 413, the tip dismounting judgment section 517 calculates an average value. The tip dismounting judgment section 517 compares the average value with a predetermined threshold to judge whether or not the measuring tip 308 is dismounted from the optical measuring section 103.

The tip mounting judgment section 513 and the tip dismounting judgment section 517 are functional blocks for detecting mounting/dismounting of the measuring tip 308 with respect to the optical measuring section 103. Thus, since the only thing needed to do is to judge "whether or not there is a measuring tip 308", the A/D converter 413 causes the red LED 303 to emit light using the low-speed clock to perform judgment with less sample data.

In contrast, the reference value measuring section 514, the blood spotting judgment section 515 and the measurement processing section 516 are functional blocks required for acquiring specific numerical data (i.e., the amount of reflected light in the state before the blood has been spotted onto the test paper 511, the judgment of the moment when the blood has been spotted onto the test paper 511, and the amount of reflected light which continuously changes until a predetermined time has elapsed). Thus, since high accuracy is required, the A/D converter 413 causes both the red LED 303 and the green LED 309 to emit light using high-speed clock to acquire more sample data than using low-speed clock.

FIG. 7 is a functional block diagram of the high-speed clock generator 520.

A 512 Hz clock generator 702 generates the 512 Hz clock pulses.

A loop counter 703 counts the number of the 512 Hz clock pulses from 0 to 511 (512 counts). A 256/512 switching control signal is provided from the controller 518 to the loop counter 703, and the loop counter 703 counts number from 0 to 511, or from 0 to 255 (256 counts) based on the control signal. Incidentally, when there is a signal provided to the reset terminal, the loop counter 703 continues to output 0 without performing counting operation.

The output data line of the loop counter 703 is connected to the negative side input terminal of a digital comparator 704. A numerical data "127", as a threshold 705, stored in the ROM 403 is provided to the positive side input terminal of the digital comparator 704, and the digital comparator 704 outputs the comparison result between the threshold 705 and the counted value of the loop counter 703 to an AND gate 706.

While the digital comparator 704 is outputting logical "true" (high potential), the AND gate 706 outputs the clock pulses of the 512 Hz clock generator 702.

In other words, in a state where the loop counter 703 performs count with 512 as the largest value, the digital comparator 704 outputs logical true while the loop counter 703 is counting number from 0 to 127. On the other hand, the digital comparator 704 outputs logical false while the loop counter 703 is counting number from 128 to 511. Thus, 128 pieces of clock pulses are outputted from the AND gate 706 every 1 second. The aforesaid is the operation of the high-speed clock generator 520 while the measurement processing section 516 is in operation.

Similarly, in a state where the loop counter 703 performs count with 256 as the largest value, the digital comparator 704 outputs logical true while the loop counter 703 is counting number from 0 to 127. On the other hand, the digital comparator 704 outputs logical false while the loop counter 703 is counting number from 128 to 255. Thus, 128 pieces of clock pulses are outputted from the AND gate 706 every 0.5 seconds. The aforesaid is the operation of the high-speed clock generator 520 while the reference value measuring section 514 is in operation.

Further, when the reset signal is continued to be provided to the loop counter 703, since the loop counter 703 continues to output 0, the digital comparator 704 continues to output logical true. Thus, the clock pulses of the 512 Hz clock generator 702 are continuously outputted from the AND gate 706. The aforesaid is the operation of the high-speed clock generator 520 while the blood spotting judgment section 515 is in operation.

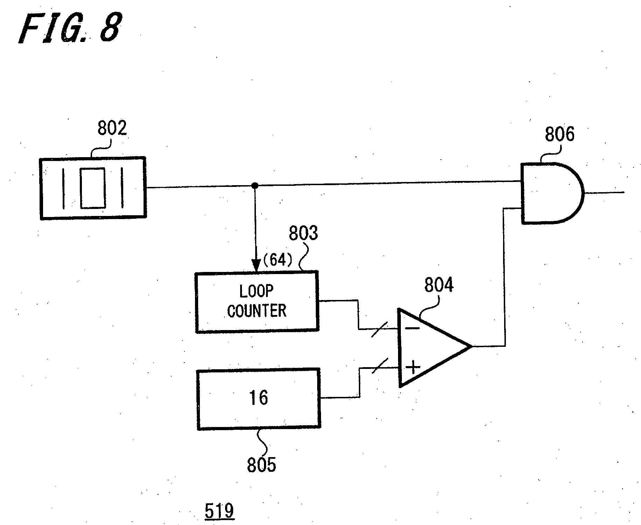

FIG. 8 is a functional block diagram of the low-speed clock generator 519.

A 64 Hz clock generator 802 generates the 64 Hz clock pulse.

A loop counter 803 counts the number of the 64 Hz clock pulses from 0 to 63 (64 counts).

The output data line of the loop counter 803 is connected to the negative side input terminal of a digital comparator 804. A numerical data "16", as a threshold 805, stored in the ROM 403 is provided to the positive side input terminal of the digital comparator 804, and the digital comparator 804 outputs the comparison result between the threshold 805 and the counted value of the loop counter 803 to an AND gate 806.

The low-speed clock generator 519 has similar circuit configuration to the high-speed clock generator 520 shown in FIG. 7 except that the low-speed clock generator 519 has different frequency of the clock, different counted value of the loop counter and different number of the threshold from the high-speed clock generator 520, and that the loop counter of the low-speed clock generator 519 has no reset terminal and counting switching control line compared with the high-speed clock generator 520.

While the digital comparator 804 is outputting logical "true" (high potential), the AND gate 806 outputs the clock pulses of the 64 Hz clock generator 802.

The digital comparator 804 outputs logical true while the loop counter 803 is counting number from 0 to 16. On the other hand, the digital comparator 804 outputs logical false while the loop counter 803 is counting number from 17 to 63. Thus, 16 pieces of clock pulses are outputted from the AND gate 806 every 1 second. The aforesaid is the operation of the low-speed clock generator 519 while the tip mounting judgment section 513 and the tip dismounting judgment section 517 are in operation.

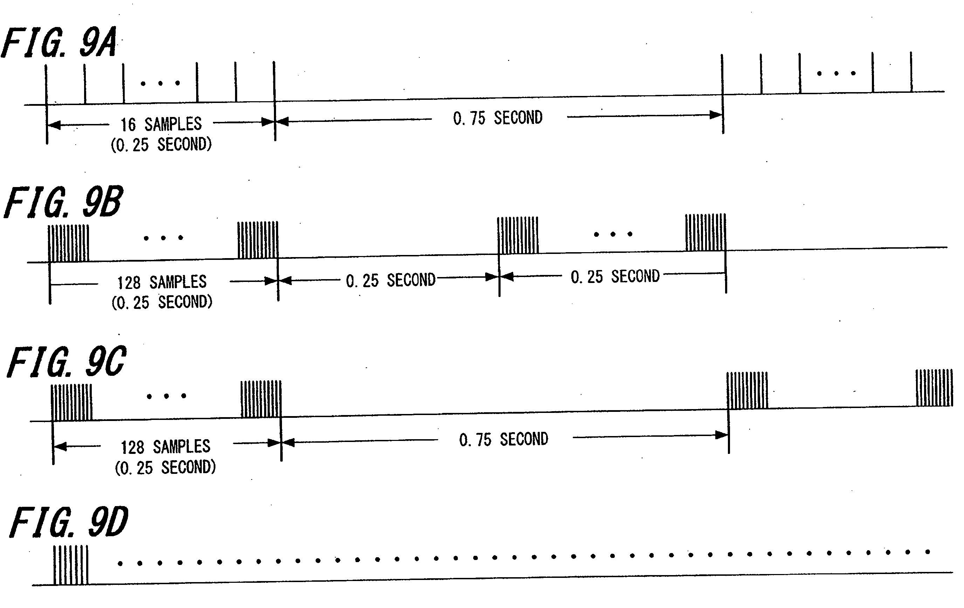

FIGS. 9A, 9B, 9C and 9D are each a timing diagram showing the clock pulses generated by the low-speed clock generator 519 or the high-speed clock generator 520.

FIG. 9A shows the clock pulses generated by the low-speed clock generator 519. 16 pieces of pulses are outputted in a duration of 0.25 seconds, and no pulse is outputted in a duration of 0.75 seconds. This process is repeated. Thus, the period is 1 second.

FIG. 9B shows the clock pulses generated by the high-speed clock generator 520 while the controller 518 is causing the reference value measuring section 514 to operate. 128 pieces of pulses are outputted in a duration of 0.25 seconds, and no pulse is outputted in a duration of 0.25 seconds. This process is repeated. Thus, the period is 0.5 seconds.

FIG. 9C shows the clock pulses generated by the high-speed clock generator 520 while the controller 518 is causing the measurement processing section 516 to operate. 128 pieces of pulses are outputted in a duration of 0.25 seconds, and no pulse is outputted in a duration of 0.75 seconds. This process is repeated. Thus, the period is 1 second.

FIG. 9D shows the clock pulses generated by the high-speed clock generator 520 while the blood spotting judgment section 515 is in operation. Similar to FIGS. 9B and 9C, a 512 Hz clock is continuously outputted.

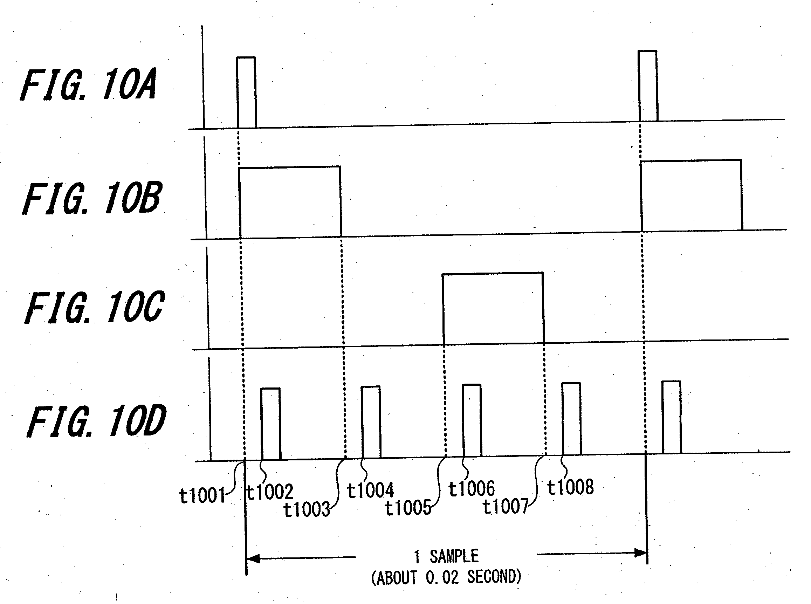

FIGS. 10A, 10B, 10C and 10D are each a timing diagram showing the timing pulses or the sampling clock generated by the high-speed sequencer 505.

FIG. 10A shows the clock pulses generated by the high-speed clock generator 520 and inputted to the high-speed sequencer 505.

FIG. 10B shows red LED emission control signals outputted by the high-speed sequencer 505 and adapted to control the emission driving of the red LED 303.

FIG. 10C shows a green LED emission control signal outputted by the high-speed sequencer 505 and adapted to control the emission driving of the green LED 309.

FIG. 10D shows the sampling clock outputted by the high-speed sequencer 505 and provided to the A/D converter 413.

The high-speed sequencer 505 starts at the rising edge of the clock pulses generated by the high-speed clock generator 520 (t1001). In response to the rising edge of the clock pulses, the high-speed sequencer 505 generates red LED emission control signals with a duration (t1001 to t1003) shown in FIG. 10B.

Next, the high-speed sequencer 505 generates the sampling clock shown in FIG. 10D at timing slightly later than the rising edge (t1001) of the red LED emission control signal (t1002). In response to this, the A/D converter 413 converts the signal voltage of the reflected light of the test paper 511 into data in a state where the red LED 303 is emitting light.

Next, the high-speed sequencer 505 generates the sampling clock shown in FIG. 10D at timing slightly later than the falling edge (t1003) of the red LED emission control signal (t1004). In response to this, the A/D converter 413 converts the signal voltage of the reflected light of the test paper 511 into data in a state where the red LED 303 is not emitting light.

Next, the high-speed sequencer 505 generates a green LED emission control signal with a duration (t1005 to t1007) shown in FIG. 10C.

Next, the high-speed sequencer 505 generates the sampling clock shown in FIG. 10D at timing slightly later than the rising edge (t1005) of the green LED emission control signal (t1006). In response to this, the A/D converter 413 converts the signal voltage of the reflected light of the test paper 511 into data in a state where the green LED 309 is emitting light.

Next, the high-speed sequencer 505 generates the sampling clock shown in FIG. 10D at timing slightly later than the falling edge (t1007) of the green LED emission control signal (t1008). In response to this, the A/D converter 413 converts the signal voltage of the reflected light of the test paper 511 into data in a state where the green LED 309 is not emitting light.

It is known from the above that, in response to the rising edge of the clock pulses generated by the high-speed clock generator 520, the high-speed sequencer 505 alternately causes the red LED 303 and the green LED 309 to emit light, and meanwhile drives the A/D converter 413 twice each time. The A/D converter 413 samples the data of the red LED 303 and the data of the green LED 309 both in the state where the red LED 303 and the green LED 309 are lighting and the state where the red LED 303 and the green LED 309 are not lighting, and' outputs the data.

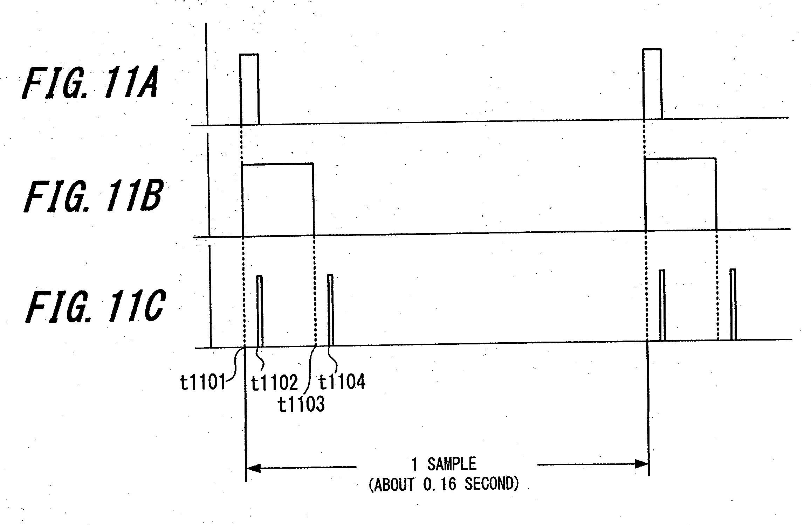

FIGS. 11A, 11B and 11C are each a timing diagram showing the timing pulses or the sampling clock generated by the low-speed sequencer 504.

FIG. 11A shows the clock pulses generated by the low-speed clock generator 519 and inputted to the low-speed sequencer 504.

FIG. 11B shows red LED emission control signals outputted by the low-speed sequencer 504 and adapted to control the emission driving of the red LED 303.

FIG. 11C shows the sampling clock outputted by the low-speed sequencer 504 and provided to the A/D converter 413.

The low-speed sequencer 504 starts at the rising edge (FIG. 11A) of the clock pulses generated by the low-speed clock generator 519 (t1101). In response to the rising edge of the clock pulses, the low-speed sequencer 504 generates a red LED emission control signal with a duration (t1101 to t1103) shown in FIG. 11B.

Next, the low-speed sequencer 504 generates the sampling clock shown in FIG. 11C at timing slightly later than the rising edge (t1101) of the red LED emission control signal (t1102). In response to this, the A/D converter 413 converts the signal voltage of the reflected light of the test paper 511 into data in a state where the red LED 303 is emitting light.

Next, the low-speed sequencer 504 generates the sampling clock shown in FIG. 11C at timing slightly later than the falling edge (t1103) of the red LED emission control signal (t1104). In response to this, the A/D converter 413 converts the signal voltage of the reflected light of the test paper 511 into data in a state where the red LED 303 is not emitting light.

It is known from the above that, in response to the rising edge of the clock pulses generated by the low-speed clock generator 519, the low-speed sequencer 504 causes the red LED 303 to emit light, and drives the A/D converter 413 twice during the period while the red LED 303 is emitting light. The A/D converter 413 samples the data of the red LED 303 both in the state where the red LED 303 is lighting and the state where the red LED 303 is not lighting, and outputs the data.

[Operation]

As described above, the blood glucose meter causes the blood to be reacted with the predetermined reagent, detects the change of the reagent, and calculates the blood glucose value. To accurately perform measurement, if the reaction time of the reaction between the glucose in the blood and the reagent is sufficient, the reaction will finally become stable, and therefore blood glucose level can be simply detected only based on the strength of the reflected light. However, since there are a lot of patients to be measured in a hospital, the blood glucose measurement in the hospital has to be performed quickly. However, in the blood glucose meter 101, by using the standard curve data, the blood glucose measurement can be performed within short time by calculating the blood glucose value in the course of the reaction between the glucose and the reagent.

In order to measure blood glucose level within short time, it is necessary for the blood glucose meter 101 to accurately acquire the timing of the spotting of the blood onto the test paper 511. If there is a difference in time axis between the acquired timing and the primary timing, the difference will appear as measurement error. The measurement error needs to be controlled within an acceptable range.

For example, the measurement time of the blood glucose meter 101 of the present embodiment is set to 9 seconds; and on this basis, if the frequency of the sampling clock is 1 Hz, an error of up to 2 second will be caused. As described above, since the blood glucose value is calculated in the course of the reaction between the glucose and the reagent, when 2 second elapses, the reaction will progress accordingly, and therefore a large error will be caused.

To reduce the error, the frequency of the sampling clock may be increased. However, increasing the frequency of the sampling clock means that the A/D converter 413 will be more operated accordingly. Generally, since the A/D converter has high power consumption, it is not preferred to set the frequency of the sampling clock to high in the case of a portable device driven by battery, because that will increase the power consumption of the battery and therefore reduce the running time.

To solve this problem, the present embodiment employs a method in which the frequency of the sampling clock is increased only in a stage when confirming the spotting of the blood onto the test paper 511. The high-speed clock generator 520 is configured so that, from the moment when the spotting of the blood onto the test paper 511 has been confirmed, the measurement processing section 516 is controlled so that the timer for performing the measurement processing is started, and the sampling clock of the A/D converter 413 is provided.

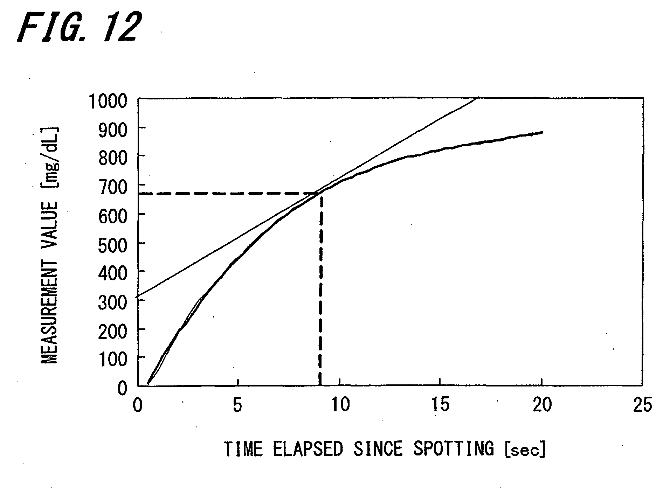

FIG. 12 is a graph showing the measurement value calculated using the standard curve data when measuring blood glucose level, with the time course. The blood glucose meter of the present embodiment acquires the values of the graph from the 0th second to the 9th second.

The measurement error depends on the slope of the graph at the 9th second, which is the time when the measurement is terminated. Considering the above, the sampling time interval (i.e., the inverse of the frequency of the sampling clock) $T_{sample}$, the slope $\Delta f$ of the graph and the required resolution $\varepsilon$ of the blood glucose meter have a relation as below.

[Formula 1]

$$T_{sample} \leq \frac{\varepsilon}{\Delta f}$$

According to the above formula, with the response curve which has different slopes corresponding to different measurement times, it is possible to obtain the maximum value of the optimal sampling time interval (i.e., the minimum value of the frequency of the clock) for each measurement time.

In the blood glucose meter 101 of the present embodiment, the minimum value of the displayed blood glucose value is 1 mg/dL. Since the resolution of an electronic measuring device is generally required to be ten times or more than the displayed value, if the required resolution is set to 0.1 mg/dL and the slope is set to 40 mg/dl/sec, the sampling time interval will be 0.0025 second and therefore the frequency of the sampling clock (i.e., the inverse of the sampling time interval) will be 400 Hz. Incidentally, for convenience of designing digital device, 512 Hz, which a number easily manageable in binary number system, is adapted as the frequency of the sampling clock of the blood glucose meter 101 of the present embodiment.

The blood glucose meter is disclosed in the present embodiment.

The clock generators are configured so that the sampling clock is suitably set according to the operating mode of the blood glucose meter. Particularly, the clock generators are configured so that the high-speed clock pulses are continuously generated in a mode that determines measurement start, and, from the time when the spotting is detected, the measurement operation of the timer and the like is started, and the mode is switched from continuously generating the clock pulses to intermittently generating the clock pulses. The clock pulse is determined based on the required resolution and the slope of the blood glucose measurement curve.

By configuring the blood glucose meter in this manner, it is possible to implement a blood glucose meter which has required measurement accuracy while reducing power consumption as much as possible

It is to be understood that, although the present invention is described based on the aforesaid embodiment, the present invention is not limited to the embodiment, and various modifications and applications can be made within the scope of the appended claims.

**Explanation of Reference Numerals**

| | |
|---|---|
| 101 | blood glucose meter |
| 102 | case |
| 103 | optical measuring section |
| 104 | eject lever |
| 105 | display panel |
| 106 | operation panel |
| 202 | circuit board |
| 203 | internal temperature thermistor |
| 256 | largest value |
| 302 | tube |
| 304 | photodiode |
| 305 | base |
| 306 | glass window |
| 307 | external temperature thermistor |
| 308 | measuring tip |
| 402 | CPU |
| 403 | ROM |
| 404 | RAM |
| 405 | bus |
| 407 | real-time clock |
| 408 | button operating portion |
| 410 | driver |
| 411 | D/A converter |
| 413 | A/D converter |
| 414 | nonvolatile storage |
| 415 | LCD display section |
| 424 | driver |
| 503 | switch |
| 504 | low-speed sequencer |
| 505 | high-speed sequencer |
| 506 | OR gate |
| 508 | switch |
| 511 | test paper |
| 512 | OR gate |
| 513 | tip mounting judgment section |
| 514 | reference value measuring section |
| 515 | blood spotting judgment section |
| 516 | measurement processing section |
| 517 | tip dismounting judgment section |
| 518 | controller |
| 519 | low-speed clock generator |
| 520 | high-speed clock generator |
| R502, R507, R510 | voltage-dividing resistor |
| 702 | 512 Hz clock generator |
| 703 | loop counter |
| 704 | digital comparator |
| 705 | threshold |
| 706 | AND gate |
| 802 | 64 Hz clock generator |
| 803 | loop counter |
| 804 | digital comparator |
| 805 | threshold |
| 806 | AND gate |

**Claims**

1. A blood glucose meter (101) comprising:

a blood glucose measuring section (103) adapted to, in a state where a measuring tip (308) is mounted, detect blood spotted onto the measuring tip (308), and output a signal corresponding to glucose level of the blood;
an A/D converter (413) adapted to convert the signal into digital data;
a blood spotting judgment section (515) adapted to judge whether or not the blood has been spotted onto the measuring tip (308) based on the digital data;
a measurement processing section (516) adapted to acquire a blood glucose value based on the digital data; and
a clock generator (519, 520) adapted to generate a clock for acquiring the digital data from the A/D converter (413),
**characterized in that**
the period of the clock is equal to or less than a value obtained by dividing the required resolution by the slope of a blood glucose reaction curve at a predetermined time.

2. The blood glucose meter (101) according to claim 1, wherein the blood glucose measuring section (103) has a light-emitting element (303, 309) adapted to radiate light onto the measuring tip (308), and a light-receiving element (304) connected to the A/D converter (413) and adapted to receive the reflected light obtained from the measuring tip (308) and convert the received light into an electrical signal,

wherein the clock generator (519, 520) is adapted to continuously output the clock until the blood spotting judgment section (515) judges that the blood has been spotted onto the measuring tip (308), and intermittently output the clock when the blood spotting judgment section (515) judges that blood has been spotted onto the measuring tip (308), and wherein the measurement processing section (516) is adapted to start counting a predetermined time necessary for performing blood glucose measurement processing when the blood spotting judgment section (515) judges that the blood has been spotted onto the measuring tip (308).

3. The blood glucose meter (101) according to claim 2, further comprising:

a tip mounting judgment section (513) adapted to judge whether or not the measuring tip (308) has been mounted on the blood glucose measuring section (103);
a reference value measuring section (514) adapted to, when the tip mounting judgment section (513) judges that the measuring tip (308) has been mounted on the blood glucose measuring section (103), acquire the digital data, as a reference value, from the A/D converter (413) in a state where the blood has not been spotted onto the measuring tip (308); and
a tip dismounting judgment section (517) adapted to judge whether or not the measuring tip (308) has been dismounted from the blood glucose measuring section (103) after the measurement processing section (516) has terminated the measurement processing,
wherein the blood spotting judgment section (515) is executed after the reference value measuring section (514) has acquired reference value.

**Patentansprüche**

1. Blutzuckermessvorrichtung (101), mit
einem Blutzuckermessabschnitt (103), der dazu eingerichtet ist, um, in einem Zustand, in dem eine Messspitze (308) montiert ist, auf die Messspitze (308) aufgetupftes Blut zu erfassen, und um ein Signal entsprechend einem Glukosepegel des Bluts auszugeben,
einer A/D-Wandlereinrichtung (413), die dazu eingerichtet ist, um das Signal in digitale Daten umzuwandeln,
einem Bluttupfbeurteilungsabschnitt (515), der dazu eingerichtet ist, um basierend auf den digitalen Daten zu beurteilen, ob das Blut auf die Messspitze (308) getupft wurde oder nicht,
einem Messverarbeitungsabschnitt (516), der dazu eingerichtet ist, um basierend auf den digitalen Daten einen Blutzuckerwert zu erlangen, und
einer Takterzeugungseinrichtung (519, 520), die dazu eingerichtet ist, um einen Takt für ein Erlangen der digitalen Daten von der A/D-Wandlereinrichtung (413) zu erzeugen,
**dadurch gekennzeichnet, dass**
die Periode des Takts gleich oder kleiner als ein Wert ist, der durch Teilen einer benötigten Auflösung durch eine Steigung einer Blutzuckerreaktionskurve zu einer vorbestimmten Zeit erhalten wird.

2. Blutzuckermessvorrichtung (101) nach Anspruch 1, wobei der Blutzuckermessabschnitt (103) ein Lichtabstrahlelement (303, 309), das dazu eingerichtet ist, um Licht auf die Messspitze (308) zu strahlen, und ein Lichtempfangselement (304) aufweist, das mit der A/D-Wandlereinrichtung (413) verbunden ist und dazu eingerichtet ist, um das reflektierte Licht, das von der Messspitze (308) erhalten wird, zu empfangen, und um das empfangene Licht in ein elektrisches Signal zu wandeln,
wobei die Takterzeugungseinrichtung (519, 520) dazu eingerichtet ist, um den Takt kontinuierlich auszugeben, bis der Bluttupfbeurteilungsabschnitt (515) beurteilt, dass das Blut auf die Messspitze (308) getupft wurde, und um den Takt intermittierend auszugeben, wenn der Bluttupfbeurteilungsabschnitt (515) beurteilt, dass Blut auf die Messspitze (308) getupft wurde, und
wobei der Messverarbeitungsabschnitt (516) dazu eingerichtet ist, um ein Zählen einer vorbestimmten Zeit, die benötigt ist, um eine Blutzuckermessverarbeitung durchzuführen, zu beginnen, wenn der Bluttupfbeurteilungsabschnitt (515) beurteilt, dass das Blut auf die Messspitze (308) getupft wurde.

3. Blutzuckermessvorrichtung (101) nach Anspruch 2, ferner mit
einem Spitzenmontagebeurteilungsabschnitt (513), der dazu eingerichtet ist, um zu beurteilen, ob die Messspitze (308) bei dem Blutzuckermessabschnitt (103) montiert wurde oder nicht,
einem Referenzwertmessabschnitt (514), der dazu eingerichtet ist, um, wenn der Spitzenmontageabschnitt (513) beurteilt, dass die Messspitze (308) bei dem Blutzuckermessabschnitt (103) montiert wurde, die digitalen Daten als einen Referenzwert von der A/D-Wandlereinrichtung (413) zu erlangen in einem Zustand, in dem das Blut nicht auf die Messspitze (308) getupft wurde, und
einem Spitzendemontagebeurteilungsabschnitt (517), der dazu eingerichtet ist, um zu beurteilen, ob die Messspitze (308) von dem Blutzuckermessabschnitt (103) demontiert wurde oder nicht, nachdem der Messverarbeitungsabschnitt (516) die Messverarbeitung abgeschlossen hat,
wobei der Bluttupfbeurteilungsabschnitt (515) ausgeführt wird, nachdem der Referenzwertmessabschnitt (514) einen Referenzwert erlangt hat.

**Revendications**

1. Glucomètre sanguin (101) comprenant :

une section de mesure de glucose sanguin (103) conçue pour, dans un état où une extrémité de mesure (308) est montée, détecter le sang déposé sur l'extrémité de mesure (308), et délivrer un signal correspondant au niveau de glucose du sang ;
un convertisseur analogique-numérique (413) conçu pour convertir le signal en données numériques ;
une section de jugement de dépôt de sang (515) conçue pour juger si, oui ou non, le sang a été déposé sur l'extrémité de mesure (308) sur la base des données numériques ;
une section de traitement de mesure (516) conçue pour acquérir une valeur de glucose sanguin sur la base des données numériques ; et
un générateur d'horloge (519, 520) conçu pour générer une horloge pour acquérir les données numériques à partir du convertisseur analogique-numérique (413),
**caractérisé en ce que**
la période de l'horloge est égale ou inférieure à une valeur obtenue en divisant la résolution requise par la pente de la courbe de réaction au glucose sanguin à un instant prédéterminé.

2. Glucomètre sanguin (101) selon la revendication 1, dans lequel la section de mesure de glucose sanguin (103) comporte un élément d'émission de lumière (303, 309) conçu pour rayonner une lumière sur l'extrémité de mesure (308), et un élément de réception de lumière (304) connecté au convertisseur analogique-numérique (413) et conçu pour recevoir la lumière réfléchie obtenue à partir de l'extrémité de mesure (308) et convertir la lumière reçue en un signal électrique,
dans lequel le générateur d'horloge (519, 520) est conçu pour délivrer continûment l'horloge jusqu'à ce que la section de jugement de dépôt de sang (515) juge que le sang a été déposé sur l'extrémité de mesure (308), et délivre par intermittence l'horloge lorsque la section de jugement de dépôt de sang (515) juge que le sang a été déposé sur l'extrémité de mesure (308), et
dans lequel la section de traitement de mesure (516) est conçue pour débuter le comptage d'un temps prédéterminé nécessaire pour exécuter un traitement de mesure de glucose sanguin lorsque la section de jugement de dépôt de sang (515) juge que le sang a été déposé sur l'extrémité de mesure (308).

3. Glucomètre sanguin (101) selon la revendication 2, comprenant en outre :

une section de jugement de montage d'extrémité (513) conçue pour juger si, oui ou non, l'extrémité de mesure (308) a été montée sur la section de mesure de glucose sanguin (103) ;
une section de mesure de valeur de référence (514) conçue pour, lorsque la section de jugement de montage d'extrémité (513) juge que l'extrémité de mesure (308) a été montée sur la section de mesure de glucose sanguin (103), acquérir les données numériques, en tant que valeur de référence, à partir du convertisseur analogique-numérique (413) dans un état dans lequel le sang n'a pas été déposé sur l'extrémité de mesure (308) ; et
une section de jugement de démontage d'extrémité (517) conçue pour juger si, oui ou non, l'extrémité de mesure (308) a été démontée de la section de mesure de glucose sanguin (103) après que la section de traitement de mesure (516) a terminé le traitement de mesure,
dans lequel la section de jugement de dépôt de sang (515) est exécutée après que la section de mesure de valeur de référence (514) a acquis une valeur de référence.

# FIG. 1

# FIG. 2

*FIG. 3*

103

# FIG. 4

# FIG. 5

EP 2 479 575 B1

## FIG. 6

| FUNCTIONAL BLOCK | SEQUENCER | INTERMITTENT OPERATION | CLOCK AND SAMPLE NUMBER | LED THAT EMITS LIGHT |
|---|---|---|---|---|
| TIP MOUNTING JUDGMENT SECTION | LOW-SPEED SEQUENCER | 1 SECOND | 64Hz 16 SAMPLES | RED LED ONLY |
| REFERENCE VALUE MEASURING SECTION | HIGH-SPEED SEQUENCER | 0.5 SECOND | 512Hz 128 SAMPLES | RED LED AND GREEN LED |
| SPOTTING JUDGMENT SECTION | HIGH-SPEED SEQUENCER | CONTINUOUS | 512Hz (128 SAMPLES) | RED LED AND GREEN LED |
| MEASUREMENT PROCESSING SECTION | HIGH-SPEED SEQUENCER | 1 SECOND | 512Hz 128 SAMPLES | RED LED AND GREEN LED |
| TIP DISMOUNTING JUDGMENT SECTION | LOW-SPEED SEQUENCER | 1 SECOND | 64Hz 16 SAMPLES | RED LED ONLY |

## FIG. 7

702

706

703

RESET

704

256/512 SWITCHING

LOOP COUNTER

127

705

520

# FIG. 8

802

806

803

(64)

LOOP
COUNTER

804

16

805

519

## FIG. 9A

16 SAMPLES
(0. 25 SECOND)

0. 75 SECOND

## FIG. 9B

128 SAMPLES
(0. 25 SECOND)

0. 25 SECOND

0. 25 SECOND

## FIG. 9C

128 SAMPLES
(0. 25 SECOND)

0. 75 SECOND

## FIG. 9D

FIG. 10A

FIG. 10B

FIG. 10C

FIG. 10D

t1001    t1002    t1004    t1006    t1008
        t1003    t1005    t1007

1 SAMPLE
(ABOUT 0.02 SECOND)

EP 2 479 575 B1

EP 2 479 575 B1

FIG. 11A

FIG. 11B

FIG. 11C

t1101  t1102  t1104
t1103

1 SAMPLE
(ABOUT 0.16 SECOND)

FIG. 12

**EP 2 479 575 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2004144750 A
- JP 2006071421 A
- US 2008309939 A1
- CA 2241964 A1